(19) Europäisches Patentamt

European Patent Office

Office européen des brevets

(11) Veröffentlichungsnummer: **0 253 796 B1**

(12) **EUROPÄISCHE PATENTSCHRIFT**

(45) Veröffentlichungstag der Patentschrift: **02.12.92**

(51) Int. Cl.⁵: **A61B 17/58**

(21) Anmeldenummer: **87890155.2**

(22) Anmeldetag: **06.07.87**

(54) **Anatomische Knochenplatte bzw. Fixationsplatte.**

(30) Priorität: **15.07.86 AT 1927/86**
**02.12.86 AT 3215/86**

(43) Veröffentlichungstag der Anmeldung:
**20.01.88 Patentblatt 88/03**

(45) Bekanntmachung des Hinweises auf die
Patenterteilung:
**02.12.92 Patentblatt 92/49**

(84) Benannte Vertragsstaaten:
**BE CH DE ES FR GB GR IT LI LU NL SE**

(56) Entgegenhaltungen:
**EP-A- 0 100 114**
**DE-B- 2 410 057**
**GB-A- 2 125 295**
**US-A- 4 454 876**

(73) Patentinhaber: **Forschungsstelle für Hüftdys-**
**plasie Gesellschaft m.b.H.**
**Margaretenstrasse 2-4**
**A-1040 Wien(AT)**

(72) Erfinder: **David, Thomas, Dr.**
**Margaretenstrasse 2-4**
**A-1040 Wien(AT)**
Erfinder: **Uyka, Dieter, Dipl.-Ing.**
**Ziegelhofstrasse 32-34**
**A-1220 Wien(AT)**

(74) Vertreter: **Collin, Hans, Dipl.-Ing. Dr. et al**
**Patentanwälte Dipl.-Ing. Dr. Hans Collin**
**Dipl.-Ing. Erwin Buresch Dipl.-Ing. Dr. Helmut**
**Wildhack Dipl.-Ing. Armin Häupl Mariahilfer**
**Strasse 50**
**A-1070 Wien(AT)**

## Beschreibung

Die Erfindung betrifft eine Knochenfixationsplatte zur Schraubfixierung des Pfannenfragments am Darmbein nach Beckenosteotomien mit Pfannendachschwenkung beim Menschen. Insbesondere ist die Platte für Beckenosteotomien vorgesehen, bei der die Pfanne im Bereich des Darm- und Sitzbeines mit einem gegebenenfalls gemeinsam angelegten Schnitt, insbesondere mit im wesentlichen entlang eines Zylindermantels erfolgender Schnittführung, und im Bereich des Schambeines mit einem gesonderten, insbesondere ebenen Schnitt umtrennt wird, wobei die stabile Fixation dieser Platte an der Facies glutaea des cranialen Beckenpfeilers der Darmbeinschaufel erfolgt.

Es gibt verschiedene Techniken der Zweifach- und Dreifach-Osteotomien für die Hüftpfannenschwenkung bei Hüftgelenksdysplasien, insbesondere im Humanbereich. Als erfolgversprechendste ist eine Dreifach-Osteotomie anzusehen, bei der die Pfanne pfannennahe umtrennt wird, weil sich danach die Pfanne sowohl nach lateral als auch nach ventral neigen läßt. Dazu ist eine Trennung am Darmbein, am Sitzbein und am Schambein erforderlich. Zuerst wird die Sitzbein-Osteotomie durchgeführt, wobei vom Foramen ischiadicum zum Foramen obturatum durchtrennt wird, das Sitzbein dorsal aber mit Tuber, Spina und den zum Kreuzbein ziehenden Bändern erhalten bleibt. Hiebei ist zu beachten, daß das Foramen obturatum wirklich getroffen wird, wobei auch die durch den Umstand, daß das Sitzbein zur Membrana obturata spitz ausgezogen ist, gebildete Knochenleiste durchtrennt werden muß, damit letztlich die Hüftpfanne gedreht werden kann.

Anschließend wird die Osteotomie des Schambeins dicht neben dem Hüftgelenk durchgeführt. Sie wird annähernd parallel zur Körperachse in leicht schräger Richtung in das Foramen obturatum gelegt, um zu erzielen, daß nach der Pfannendachschwenkung noch Knochenkontakt erhalten bleibt, um eine rasche anschließende Knochendurchbauung zu ermöglichen.

Als dritte Stufe erfolgt die Darmbein-Osteotomie. Vorher wird ein Steinmann-Nagel als spätere Hilfe beim Verdrehen und Schwenken der Pfanne, parallel zur geplanten, schräg nach medial absteigenden Osteotomie in den Beckenknochen eingeschossen. Die Darmbein-Osteotomie erfolgt in einer Ebene, zuerst mit der oszillierenden Säge, dann mittels Meißeln.

Anschließend wird die Pfanne über den Hüftkopf verdreht und verschwenkt; jedenfalls in dem Ausmaß, in dem vorher der Hüftkopf abgespreizt werden mußte, um unter das Pfannendach zu kommen. Dazu wird das Pfannendoch am Steinmann-Nagel über den Hüftkopf und bei Bedarf auch nach vorne gezogen, sowie der mediale Schambeinast im Rotationssinne nach aufwärts gedrückt. Anschließend wird die Pfanne durch händischen Druck medialisiert und ein guter Kontakt mit dem Schambein hergestellt.

Es soll eine weitgehende horizontale Überdachung des Kopfes durch die Sklerosierungszone des Pfannendaches bzw. die Facies lunata erzielt werden, da dies die geeignete Fläche zur Aufnahme und Übertragung durch Belastung des Beins erzeugter Druckkräfte ist. Die anschließende Fixierung der Pfanne erfolgt über durch die Beckenschaufel in vier verschiedenen Richtungen in das Pfannenfragment eingeschossene Kirschnerdrähte, deren Hinterenden am Beckenkamm abgezwickt und umgebogen werden. Diese Art der Fixation ist schwierig, oftmals wenig stabil und führt in vielen Fällen nicht zum gewünschten Erfolg, d.h. die Pfanne wackelt, die Knochendurchbauung ist stark verzögert und der Patient muß sich mit einer mehrwöchigen Liegezeit im Körperbeckengips abfinden.

Ein wesentlicher Grund dafür ist die instabile Fixation über Kirschnerdrähte, die aber nicht zuletzt deswegen nötig ist, weil die Darmbein-Osteotomie als ebener Schnitt erfolgt, somit bei Verschwenken der Pfanne nach ventral und lateral ein offener Keilspalt zwischen Darmbeinschaufel und Pfannenfragment entsteht, der in aller Regel durch einen aus dem Beckenkamm herausgesägten Knochenkeil, der in diesen Spalt implantiert wird, ausgefüllt werden muß, so daß das so entstandene Dreikörpersystem fixiert und darüber hinaus auch von der Knochendurchbauung erfaßt werden muß.

Es wurde nunmehr gefunden, daß man nach Dreifach-Osteotomien die Pfanne stabil über eine besonders ausgebildete Platte an der Facies glutaea des cranialen Beckenpfeilers der Darmbeinschaufel fixieren kann, d.h. auf die Anwendung von Kirschnerdrähten und die Keilentnahme aus der Darmbeinschaufel bzw. die Knochenkeilinterposition nicht mehr angewiesen ist.

Diese erfindungsgemäße anatomische Knochenplatte ist dadurch gekennzeichnet, daß die Platte einen proximalen Abschnitt zur im wesentlichen formschlüssigen Anlage an der Darmbeinschaufel und einen distalen Abschnitt zur im wesentlichen formschlüssigen Anlage Pfannenfragment aufweist, wobei die Abschnitte in einem Winkel zueinander stehen, der im Bereich von 10 bis 50°, bevorzugt bei etwa 30°, liegt, und zur Bildung dieses Winkels zwischen den beiden Abschnitten eine in Draufsicht keilförmige Übergangszone zur Anlage an der Osteotomiefläche des Pfannenfragments und/oder zur Anlage an der Osteotomiefläche des Darmbeins ausgebildet ist.

Dabei ist vorzugsweise die größte Breite der Übergangszone am ventralen Rand der Platte gelegen.

Osteotomieplatten, die Keilzonen aufweisen, sind aus der EP-A 0 100 114 bekannt. Diese Druckschrift offenbart Osteotomieplatten zur Anwendung bei Derotationsosteotomien, vor allem zur Behebung von Tibiarotationsanomalien. Diese bekannten Platten zeigen Auflageflächen zur Befestigung an den Knochenteilen, die in einem Winkel von 10 bis 40° zueinander stehen. Dabei sind als Übergang zu einem zentralen Plattenabschnitt jeweils Keilflächen vorgesehen. Der zentrale Plattenabschnitt ist gegenüber den Auflageflächen etwas zurückversetzt, um die zu verbindenden Knochenteile im Berührungsbereich ungehindert zentrieren zu können.

Bei den bekannten Osteotomieplatten haben weder der zurückgesetzte zentrale Plattenabschnitt noch die Keilzonen eine direkt knochenabstützende Funktion im Gegensatz zur erfindungsgemäß vorgesehenen keilförmigen Übergangszone.

Die erfindungsgemäße anatomische Fixationsplatte wird mit der Darmbeinschaufel und dem Pfannenfragment verschraubt und stützt bei einer Ausführungsform mit ihrer Übergangszone den Osteotomierand des Pfannenfragments, der infolge der Verlagerung des Pfannenfragments, insbesondere des Schwenkens nach ventral und des Kippens nach lateral, über die Darmbeinschaufelaußenfläche vorsteht, nach proximal ab, d.h. gegenüber dem Belastungsdruck am Bein.

In einem ganz anderen Zusammenhang, nämlich zur Bildung eines künstlichen Pfannendachrandes bei sonst unverändertem Hüftgelenk, also ohne Beckenosteotomie, ist aus der DE-AS 24 10 057 eine im Pfannenbereich anbringbare Knochenplatte bekannt geworden. Die Fixierung der bekannten Platte erfolgt am Darmbein durch zwei proximale Laschen, zwischen denen eine Ausnehmung zur Aufnahme der Beckenkammbasis vorgesehen ist; die Platte liegt am Pfannendach auf und ihr nach ventral eingebogener distaler Rand bildet den Pfannendachrand, mit anderen Worten wird der proximale Bereich der Pfanne - das Pfannendach - nach vorwiegend lateral künstlich verbreitert, um eine Luxation des Femurkopfs nach proximal zu verhindern. Der Zweck dieser bekannten Platte und demzufolge auch ihre geometrische Ausbildung ist von denen der erfindungsgemäßen Platte völlig verschieden.

Bei Anwendung der erfindungsgemäßen anatomischen Knochenplatte ist insbesondere eine stabile Fixation durch Kompressionsosteosynthese zwischen dem Pfannenfragment und der Darmbeinschaufel und eine frühe Belastbarkeit des Hüftgelenkes und der Extremität erzielbar, es handelt sich somit um eine primär belastungs- und übungsstabile Fixierung. Das Ergebnis ist eine rasche postoperative Mobilisierung der Patienten.

Die stabile Fixation der Platte an der Darmbeinschaufel erfolgt vorteilhaft so, daß damit der optimale Druckaufnahmebereich in der Darmbeinschaufel mit Bezug auf die O-Stellung des Femur erfaßt ist, also am Vorderteil der Ala Ossis ilii, knapp distal vom Tuberculum glutaeum, beginnend etwa einfingerbreit dorsal von der Spina iliaca anterior superior und der Spina iliaca anterior inferior und etwa bis zur Linea glutaea anterior, besonders in dessen ventralem Bereich zwischen den tragenden Teilen des kranialen und kaudalen Beckenpfeilers.

Ein wesentlicher Vorteil ergibt sich, wenn die zur Pfannendachschwenkung nötige Darmbein-Osteotomie nicht wie bisher als ebener Schnitt erfolgt, so daß nach dem Verschwenken des Pfannenfragments nach ventral und lateral durch insbesondere im wesentlichen einem Zylindermantel folgende Osteotomieflächen zwischen Pfannenfragment, Darm- und Sitzbein der - für die biomechanische Lastübertragung vom Femur über die Pfanne in das Darmbein, sowie für die postoperative Knochendurchbauung - so wichtige Formschluß zwischen Pfannenfragment, Darmbeinschaufel und Sitzbein erzielt wird.

Diese Schnittführung, welche zur Einhaltung der Kongruenzbedingung, zur Erzielung des Formschlusses zwischen Pfannenfragment und Darmbeinschaufel an die Incisura ischiadica major herangeführt wird, in Verbindung mit dem Einsatz der erfindungsgemäßen anatomisch formschlüssigen Knochenplatte (Fixationsplatte) ergibt die direkte, sowie übungs- und belastungsstabile Kompressionsosteosynthese zwischen Darmbein und Pfannenfragment ohne Knochenkeilinterposition und damit die praktisch unmittelbare Wiederbelastbarkeit des Hüftgelenkes und des Beins; in Verbindung mit einer ungehinderten Knochendurchbauung - ohne der sonst üblichen mehrwöchigen Ruhigstellung mittels Körperbeckengipsverband - ist eine rasche postoperative Mobilisierung möglich.

Diese Schnittführung, welche in einem Abstand von mindestens 10 mm, vorzugsweise in einem größeren Abstand,vom Pfannenrand geführt wird, sichert darüber hinaus auch den formschlüssigen Knochenkontakt zwischen Pfannenfragment und Sitzbein, ohne den Beckenraum medial einzuengen.

Die Schambein -Osteotomie wird - wie die Sitzbeinosteotomie - vorteilhaft als ebener Schnitt durchgeführt, wobei die Schnittebene annähernd senkrecht zur Crista obturatoria steht. Diese Schnittführung sichert letztendlich auch den bündigen Knochenkontakt zwischen dem Pfannenfragment und dem Schambein nach der Pfannendachverlagerung.

Die Ausbildung der erfindungsgemäßen anatomischen Knochenfixationsplatte zum Formschluß mit der Darmbeinschaufel und dem Pfannenfragment erfordert natürlich verschiedene Plattenprofile für die linke bzw. rechte Seite des Beckens, sowie eine Anpassung an die Beckenoberfläche. Die Platten sind in ihrer Form und Größe den sich nach der Pfannendachverlagerung ergebenden proximalen und distalen Auflagebereichen so formschlüssig angepaßt, daß sie intraoperativ keiner Formkorrektur bedürfen. Auch der Winkel zwischen dem proximalen und dem distalen Plattenabschnitt wird auf die Indikation und die chirurgischen Bedürfnisse abgestellt, so daß eine Anpassung an die Größe und Art der Pfannendachverlagerung möglich ist.

Hiebei kann auch der Fall eintreten, daß das Pfannenfragment nach seiner Verlagerung, insbesondere nach Schwenken nach ventral und Kippen nach lateral, aus biomechanischen Gründen noch als Ganzes medialisiert werden muß, beispielsweise um Distanzen zwischen 4 und 10 mm. Dies hat zur Folge, daß das Pfannenfragment in der Endlage mit seinem Osteotomierand nicht mehr oder nur teilweise nach lateral über die Darmbeinaußenfläche vorsteht, so daß es angezeigt ist, nicht den Osteotomierand des Pfannendachfragments an der Obergangszone der Fixationsplatte, sondern die Übergangszone der Fixationsplatte im dorsalen Bereich an der Osteotomiefläche des Darmbeins, jeweils nach proximal abzustützen.

Der Fall, wo es angezeigt ist, beide Abstützungsmöglichkeiten zu verwirklichen, ist z.B. dann gegeben, wenn das Pfannendachfragment dorsal nach medial und ventral nach lateral verschwenkt wird, so daß die Osteotomiefläche des Pfannenfragments im Ventralbereich über die Außenfläche des Darmbeins vorspringt und im Dorsalbereich unter die Osteotomiefläche des Darmbeins einspringt.

Nach einem weiteren Kennzeichen der erfindungsgemäßen Fixationsplatte schließt somit an eine im wesentlichen keilförmige Übergangszone zur Anlage an der Osteotomiefläche des Pfannenfragments dorsal eine im wesentlichen keilförmige Übergangszone zur Anlage an der Osteotomiefläche des Darmbeins an.

Insbesondere ist die erfindungsgemäße anatomische Knochenfixationsplatte so ausgebildet, daß die Abschnitte der Platte an der dem Pfannenfragment zugewandten Seite zur Übergangszone hin jeweils eine der Osteotomieführung angepaßte Kante, vorzugsweise eine Kante mit im wesentlichen Kreisbogenabschnittform (dessen Radius von der Größe des menschlichen Patienten abhängt) aufweisen. Die erfindungsgemäße Platte ist entsprechend der Ausbildung der Knochenoberflächen im Auflagebereich vorzugsweise in allen drei Ebenen des Raums gekrümmt.

Wie bereits erwähnt, wird die erfindungsgemäße Platte mit der Darmbeinschaufel und dem Pfannenfragment unter Kompression mit Kortikalisschrauben, verschraubt. Zu diesem Zweck ist die erfindungsgemäße Platte proximal und distal mit exzentrischen Schraubensitzen zum Aneinanderziehen von Darmbeinschaufel und Pfannendach nach dem Zugschraubenprinzip versehen.

Es wird angestrebt, daß die mit den Knochen in Berührung kommenden Bereiche der erfindungsgemäßen anatomischen Knochenfixationsplatte den Konturen der Facies glutaea entsprechend zum flächigen Anliegen ausgebildet sind, also mit der Knochenoberflächenform eng korrespondieren.

Die erfindungsgemäße Knochenfixationsplatte kann bei Patienten zwischen 15 und 45 Jahren problemlos eingesetzt werden.

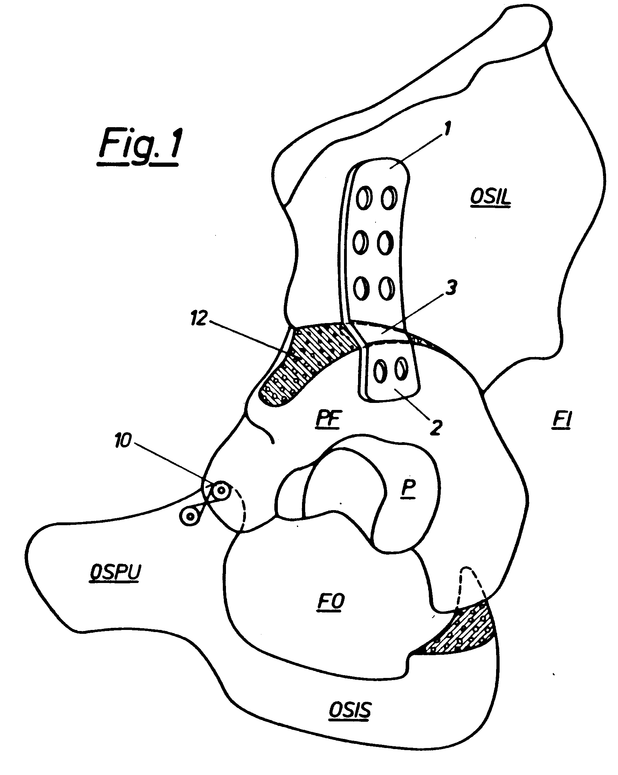

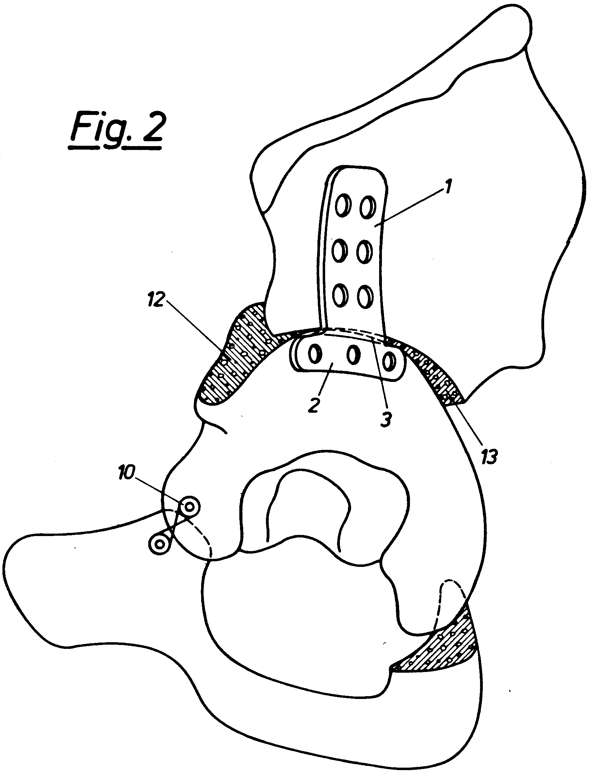

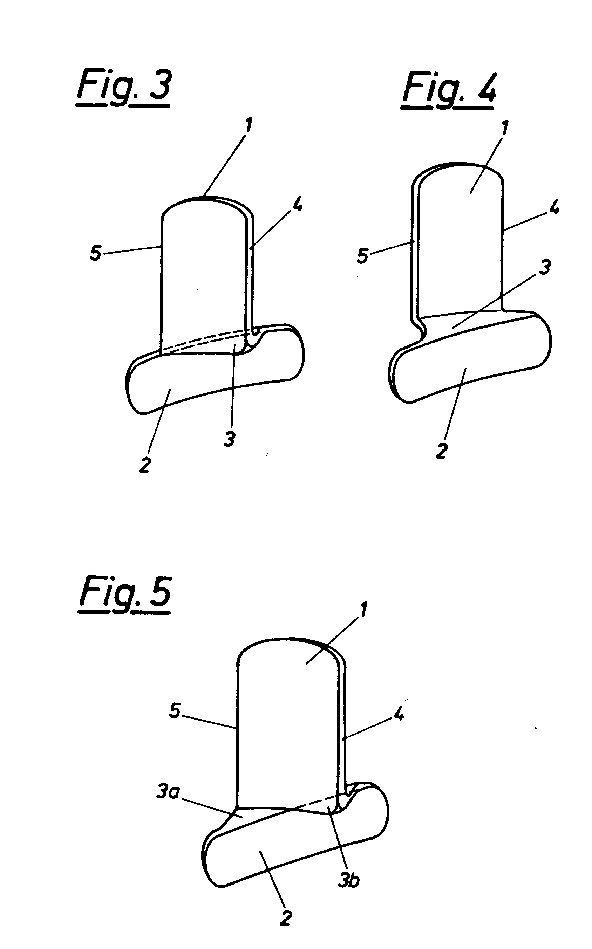

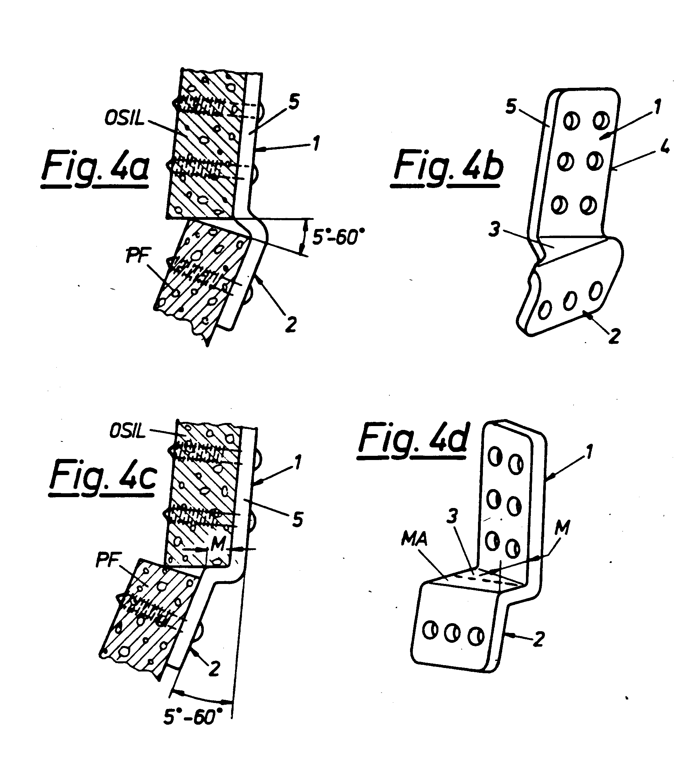

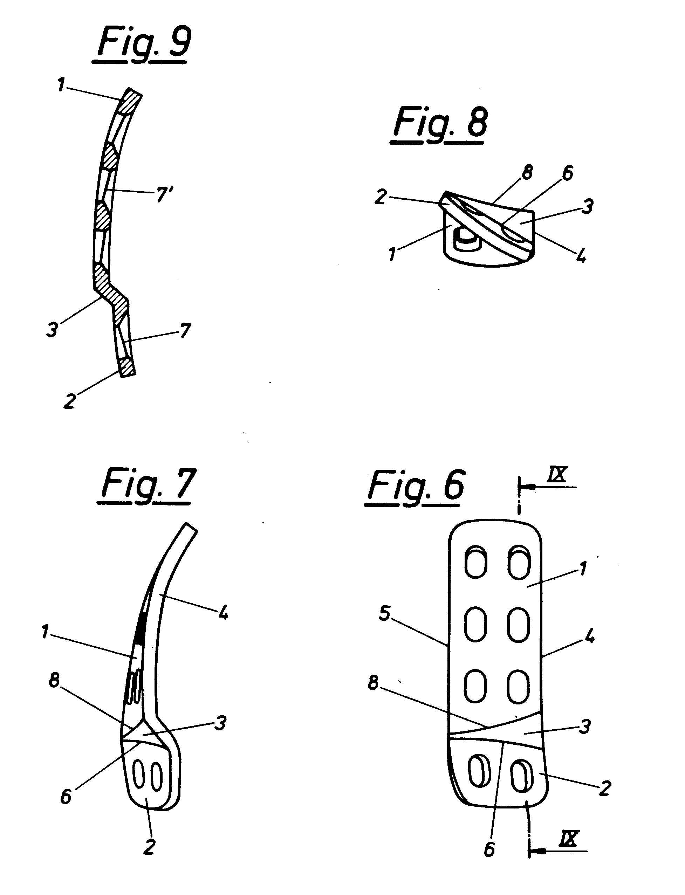

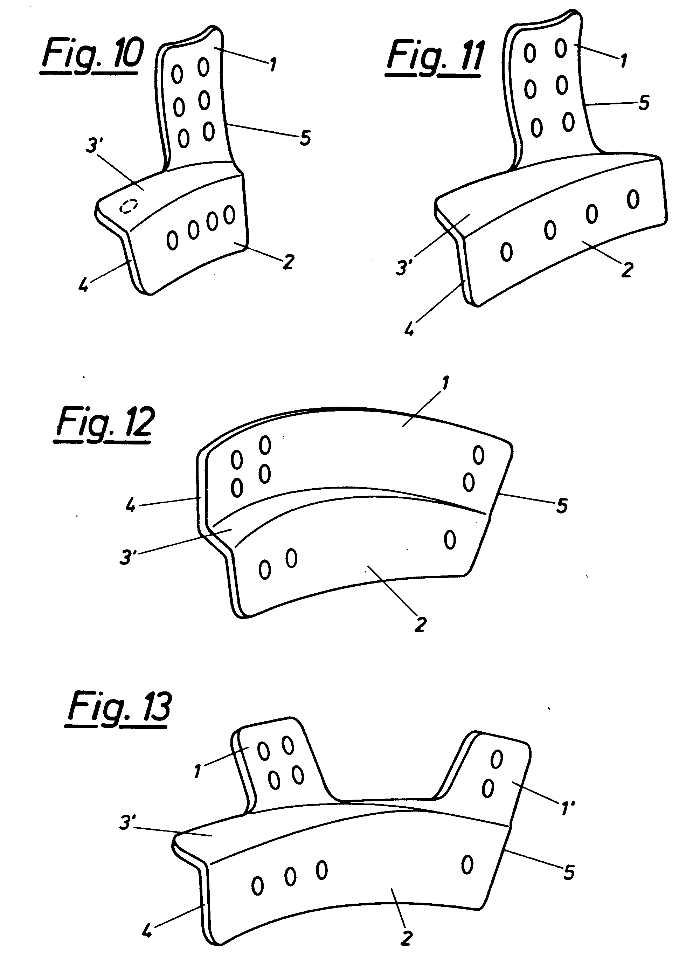

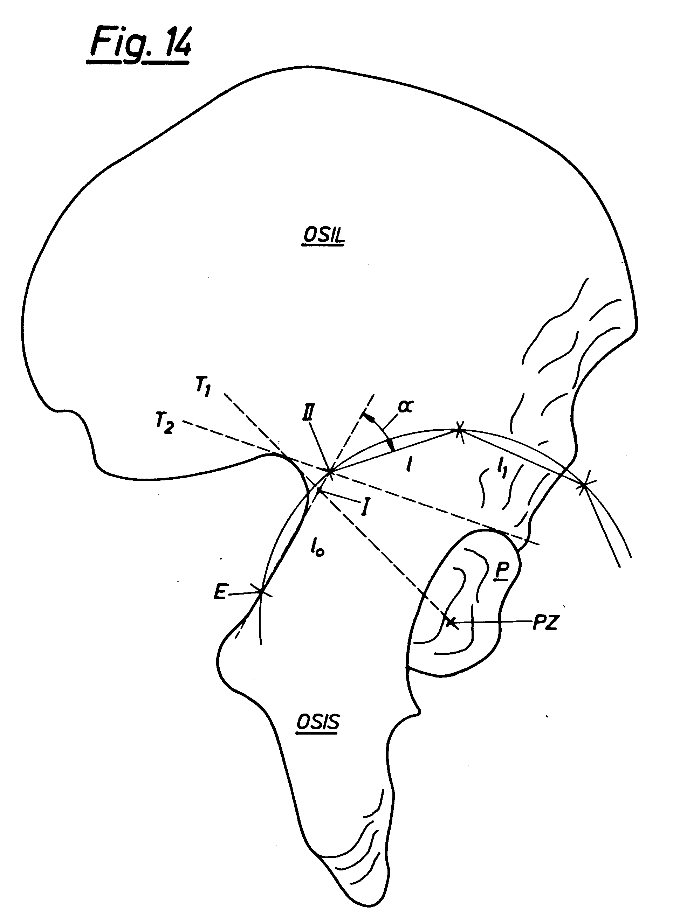

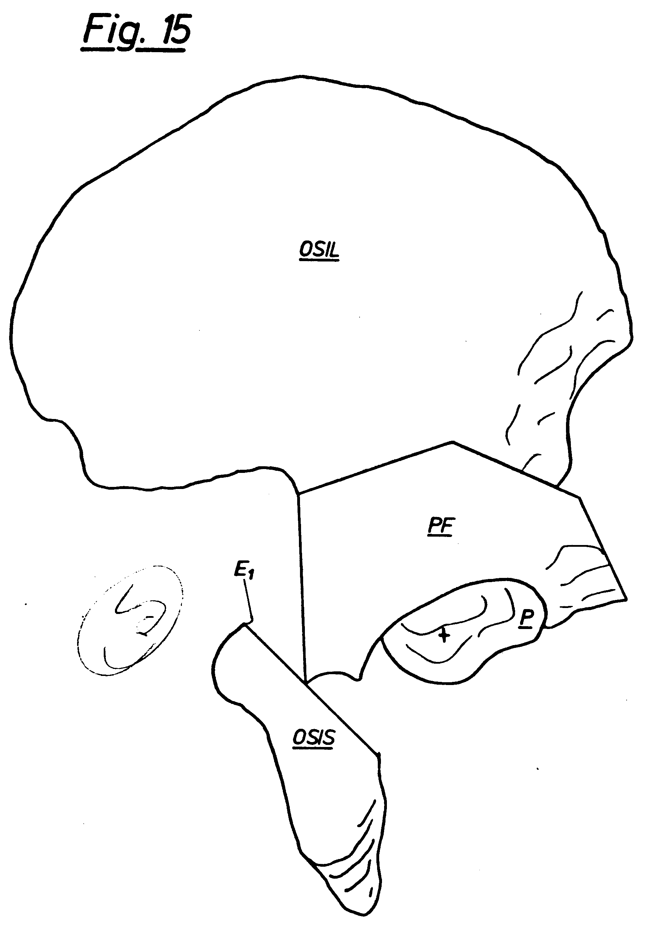

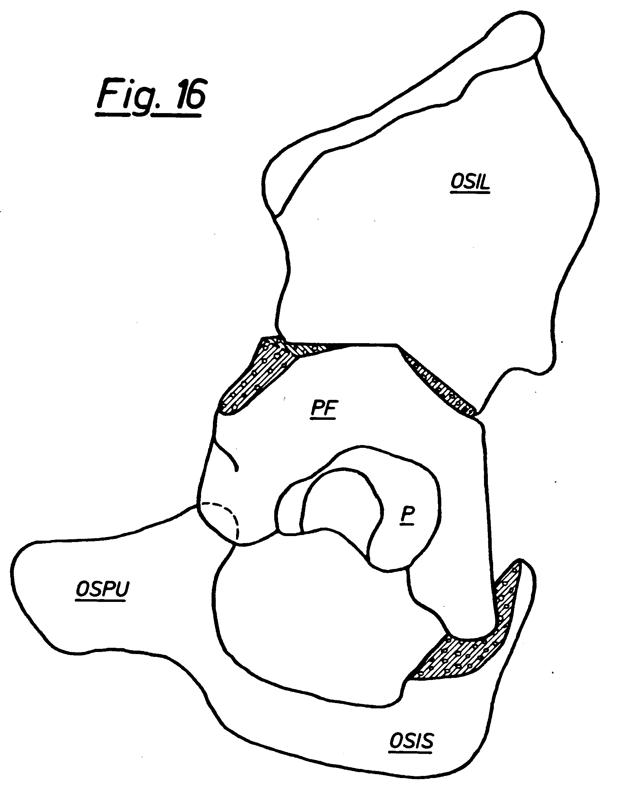

Die Erfindung wird im folgenden an Hand verschiedener Ausführungsbeispiele von erfindungsgemäßen Platten unter Bezugnahme auf die Zeichnung näher erläutert, in der die Fig. 1 und 2 schaubildlich eine Pfannenfragmentfixierung beim Menschen, Fig. 3 eine schaubildliche, schematische Ansicht einer Plattenform zur Anwendung gemäß Fig. 1 und Fig. 4 eine entsprechende Ansicht einer Plattenform zur Anwendung gemäß Fig. 2 zeigt; Fig. 4a, 4b, 4c und 4d zeigen vorteilhafte Plattenformen. Fig. 5 zeigt eine Plattenform, bei der an eine im wesentlichen keilförmige Übergangszone zur Anlage an der Osteotomiefläche des Pfannenfragments dorsal eine im wesentlichen keilförmige Übergangszone zur Anlage an der Osteotomiefläche des Darmbeins anschließt; die Fig. 6 bis 9 zeigen Details einer Plattenform des in Fig. 3 gezeigten Typs und die Fig. 10 bis 13 Varianten davon; Fig. 14 zeigt eine Röntgenprojektion des Pfannenbereichs mit einer Konstruktion zur Ermittlung eines polygonalen Osteotomieverlaufs im Darmbein; Fig. 15 ist eine Röntgenprojektion gemäß Fig. 14 nach erfolgten Osteotomien und Pfannenfragmentverschwenkung und Fig. 16 eine schaubildliche Ansicht der Situation gemäß Fig. 15.

Es sind jeweils einerseits der linke Beckenabschnitt und anderseits entsprechende linksseitige Fixationsplatten dargestellt.

Fig. 1 ist eine Ventrolateralansicht einer linksseitigen Pfannendachfixierung nach erfolgter Dreifachosteotomie mit Pfannendachverlagerung nach ventral und lateral. Durch die Pfannendachverlagerung tritt ein Teil der Osteotomiefläche 12 (strichlierter Bereich) am Pfannenfragment PF über die Außenfläche des Darmbeins (Os ilium, in der Zeichnung OSIL) nach ventrolateral vor. Am Pfannenfragment PF ist die Pfanne mit P bezeichnet. Das Foramen obturatum und das Foramen ischiadicum ist mit FO bzw. FI bezeichnet.

Man erkennt, daß die Darmbeinosteotomie im wesentlichen als Zylinderschnitt geführt wurde, d.h. die Osteotomieflächen am Pfannenfragment und Darmbein sind etwa Zylindermantelflächen. Vorzugsweise wird je nach den operativen Gegebenheiten angestrebt, sogar eine sphärische Osteotomie anzubringen, da dies die maximale Kongruenz nach Pfannendachverlagerung ergibt. Durch die Fixationsplatte 1,2,3, die im ventralen Bereich der Darmbeinschaufel (Ala ossis ilii) angebracht ist, werden Pfannenfragment PF und Darmbein an der Osteotomiefläche zusammengespannt, wobei der überstehende Osteotomierand des Pfannenfragments von der Übergangszone 3 der Platte nach proximal abgestützt wird.

Am Sitzbein (Os ischii, in der Zeichnung OSIS) erkennt man die Osteotomiefläche als strichlierten Bereich;

Die Fixierung zwischen Schambein (Os pubis, in der Zeichnung OSPU) und Pfannenfragment (PF) ist als Drahtcerclage über zwei Schrauben dargestellt.

Wie aus Fig. 1 ersichtlich, ist der proximale Abschnitt 1 der Fixationsplatte mit dem ventralen Abschnitt der Darmbeinschaufel und der distale Abschnitt 2 der Fixationsplatte mit dem Pfannenfragment verschraubt. Zwischen den beiden Abschnitten 1 und 2 der Fixationsplatte ist eine Übergangszone 3 angeordnet, die einen sich nach ventral öffnenden Keil bildet und die einen Teil des über die Darmbeinfläche nach ventrolateral vorstehenden Abschnitts der Osteotomiefläche 12 am Pfannenfragment nach proximal abstützt. Die Keilzone 3 bildet gewissermaßen eine Verbreiterung des Darmbeins nach lateral. Die größte Breite der Keilzone liegt am ventralen Rand der Platte.

Fig. 2 ist eine Fig. 1 entsprechende Darstellung, wobei aber das Pfannenfragment indikationsgemäß weitgehend medialisiert wurde, so daß die Osteotomiefläche 12 des Pfannenfragments nicht mehr über die Außenfläche des Darmbeins nach ventrolateral vorspringt, sondern im ventralen Bereich des Darmbeins etwa mit dieser fluchtet aber nach dorsal zunehmend unter die Osteotomiefläche 13 des Darmbeins nach medial einspringt. Man erkennt aus Fig. 2, daß die Fixationsplatte - ebenso wie gemäß Fig. 1 - den räumlichen Verhältnissen Rechnung trägt und im Plattenbereich an die jeweiligen Oberflächen zur Stützung angeformt ist, wobei die keilförmige Übergangszone 3 sich hier nach dorsal verbreitert, die Osteotomiefläche 13 am Darmbein untergreift und sozusagen eine laterale Verbreiterung der Osteotomiefläche 12 des Pfannenfragments zu deren Abstützung nach proximal am Darmbein bildet. Das Schambein ist zur Ermöglichung der Medialisierung und gegebenenfalls einer Kippung nach lateral entsprechend ostektomiert.

In Fig. 3 und Fig. 4 sind schaubildlich im Medioproximalsicht Fixationsplatten entsprechend den Verhältnissen in Fig. 1 und 2 zum Vergleich schematisch nebeneinandergestellt, um die beschriebenen Unterschiede deutlich zu machen, wobei jeweils 4 der ventrale Rand und 5 der dorsale Rand ist. Man erkennt, daß die Platte nach Fig. 3 für die Situation gemäß Fig. 2 und die Platte nach Fig. 4 für die Situation nach Fig. 1 zur Anwendung kommt.

Fig. 4a und 4b zeigen einen Schnitt und eine Ansicht einer Fixationsplatte insbesondere für einen Menschen, wobei das Pfannenfragment PF gegenüber dem Darmbein OSIL gedreht,geschwenkt und nach außen gekippt ist. Der Kippwinkel beträgt zwischen 5-60°. Der distale Abschnitt 2 ist gegenüber dem proximalen Abschnitt 1 um 5-60° geneigt.

Fig. 4c und 4d zeigen einen Schnitt und eine Ansicht einer Fixationsplatte insbesondere für einen Menschen, wobei das Pfannenfragment PF gegenüber dem Darmbein OSIL gedreht, geschwenkt, nach außen gekippt und medialisiert wird. Die Medialisierung M beträgt bis zu 13 mm, vorzugsweise 5-13 mm, je nach Größe des Patienten. Der Kippwinkel zwischen Darmbein OSIL und dem Pfannenfragment PF wird auf 5-60° eingestellt, die Neigung des distalen Abschnittes 2 gegenüber dem proximalen Abschnitt 1 beträgt entsprechend 5-60°.

Die Länge des ventralen Endes der Übergangszone 3 entspricht somit der notwendigen Medialisierung M. Die Übergangszone 3 erweitert sich vom ventralen Ende ausgehend nach dorsal entsprechend dem Winkel, den der proximale Abschnitt 1 und der distale Abschnitt 2 einschließen. Die Übergangszone 3 wird somit von einem Medialisierungsabschnitt MA bestimmt durch die gewünschte Medialisierung und einen keilförmigen Abschnitt aufgrund des Winkels zwischen den Abschnitten 1 und 2 gebildet. Bei dieser Fixationsplatte ist die größte Breite am dorsalen Rand gelegen.

Fig. 5 zeigt schematisch entsprechend den Ansichten von Fig. 3 und Fig. 4 eine Ausführungsform einer Fixationsplatte, bei der beide oben beschriebenen Abstützungsmöglichkeiten kombiniert sind. Die Anwendung einer derartigen Platte ist angezeigt, wenn in der zu fixierenden Endlage des Pfannenfragments die lateralen Osteotomiekanten am Darbein und am Pfannenfragment einander im Bereich der Fixationsplatte schneiden. Die Platte weist somit dorsal eine proximale Übergangskeilzone 3a und ventral eine distal Übergangskeilzone 3b auf, wobei die proximale Übergangskeilzone 3a zur abstützenden Anlage an der Osteotomiefläche 13 am Darmbein und die distale Übergangskeilzone 3b zur abstützenden Anlage an der Osteotomiefläche 12 des Pfannenfragments vorgesehen ist. Die Größe und Lage der Keilzonen 3a,3b an der Platte hängt von der gewünschten Endstellung des Pfannenfragments ab.

Die Platte nach den Fig.6 bis 9 ist räumlich gewölbt und Fig.6 zeigt eine Ansicht von medial, d.h. der Auflagefläche der Platte, Fig.7 ist eine Ansicht von ventral, Fig.8 eine Ansicht von distal und Fig.9 ein Schnitt IX-IX aus Fig. 6.

Die Platte weist einen längeren proximalen Abschnitt 1 mit sechs Schraubensitzen und einen kürzeren distalen Abschnitt 2 mit zwei Schraubensitzen auf, wobei zwischen diesen Abschnitten eine keilförmige Übergangszone 3 eingeschaltet ist, deren größte Breite am ventralen Rand 4 der Platte liegt. Ausgehend vom dorsalen Rand 5 der Platte stehen die Abschnitte 1 und 2 somit in einem Winkel gegeneinander (siehe insbesondere Fig. 8), wobei der dorsale Rand 5 praktisch in einer gleichmäßigen Wölbungslinie verläuft und im ventralen Rand 4 ein Doppelknick mit stumpfen Winkeln gegeben ist. Dies ist insbesondere aus Fig. 7 zu erkennen. Die Übergangskanten 6 und 8 zwischen dem distalen Abschnitt 2 bzw. dem proximalen Abschnitt 1 und der Übergangszone 3 weisen die Form eines Kreisbogenabschnitts auf.

In Fig. 9 erkennt man die asymmetrische Ansenkung 7,7′ der Schraubensitze, die bei den Schraubensitzen im distalen Abschnitt 2 in einer Richtung 7 und bei den Schraubensitzen im proximalen Abschnitt 1 in der anderen Richtung 7′ gegengleich geneigt sind. Die Schrauben werden dabei nicht axial in die Sitze eingesetzt, sondern im distalen Abschnitt etwas nach distal und im proximalen Abschnitt etwas nach proximal versetzt. Beim Einschrauben würde die Platte durch die Keilwirkung der Senkköpfe der Schrauben an den Keilflächen der Ansenkungen zwischen distalem Abschnitt 2 und proximalen Abschnitt 1 gedehnt werden, wenn sie nicht starr wäre. Infolge ihrer Starrheit werden statt dessen die beiden durch die Platte zu verbindenden Teile, nämlich das Darmbein, mit dem der proximale Abschnitt 1, sowie das Pfannenfragment, mit dem der distale Abschnitt 2 verschraubt wird, zusammengespannt, d.h. mit ihren Stirnflächen gegeneinander gepreßt.

Fig. 10 zeigt eine Platte mit gegenüber dem Proximalbereich 1 nach ventral verbreiterter Obergangszone 3′ und ebensolchem Distalbereich 2. Damit wird eine vollständige Abstützung des überstehenden Osteotomierandes am Pfannenfragment erzielt. Gegebenenfalls kann in der verbreiterten Übergangszone 3′ auch eine kurze Schraube nach distal in das Pfannenfragment eingebracht werden; ein entsprechender Schraubensitz ist strichliert eingezeichnet.

Fig. 11 zeigt eine ähnliche Platte wie die Platte nach Fig.10, bei der aber die Übergangszone 3′ und der Distalbereich gegenüber dem Proximalbereich 1 auch nach dorsal verbreitert sind.

Fig. 12 zeigt eine Platte, die die Darmbeinosteotomie im Bereich bis zum Foramen ischiadicum übergreift und die in den ventralen und dorsalen Endabschnitten verschraubt wird, so daß dadurch eine zusätzliche Fixierung im Bereich des Foramen ischiadicum verwirklicht ist.

Fig. 13 zeigt eine ähnliche Platte wie die Platte nach Fig. 12, wobei aber die Übergangszone 3′ und der Distalbereich 2 gegenüber dem Proximalbereich 1 nach ventral verbreitert ist, wie auch aus den Fig. 10 und 11 erkennbar. Der Mittelbereich des Proximalabschnitts 1 ist ausgeschnitten, so daß der Proximalbereich im wesentlichen aus zwei Lappen gebildet ist, wobei der ventrale Lappen 1 zur Fixierung zwischen den tragenden Teilen des cranialen und caudalen Beckenpfeilers und der dorsale Lappen 1′ zur Fixierung im Bereich des Foramen ischiadicum dient. Der Proximalbereich 1 der Platte kann auch - wie strichliert angedeutet - durchgehend ausgebildet sein, wie in der Ausführungsform nach Fig.12; ebenfalls kann bei der Ausführungsform nach Fig. 12 der Proximalabschnittmittelbereich entsprechend ausgeschnitten sein.

Die räumliche Krümmung bzw. Wölbung der Platte zur formschlüssigen Anlage an Darmbeinschaufel und Pfannenfragment ist aus den Figuren deutlich zu erkennen. So erkennt man aus Fig. 9, daß der proximale Abschnitt 1 in Proximal/Distalrichtung zur Darmbeinschaufel hin konvex und aus Fig. 8, daß der distale Abschnitt 2 in Ventral/Dorsalrichtung zum Pfannenfragment hin konkav ausgebildet ist. Aus den Fig. 7, 8, 10 und 11 erkennt man weiterhin, daß der proximale Abschnitt 1 in Ventral/Dorsalrichtung zur Darmbeinschaufel hin zusätzlich konkav gewölbt ist. Ebenso erkennt man aus den Fig. 9 und 10 bis 13, daß der distale Abschnitt 2 in Proximal/Distalrichtung zum Pfannenfragment hin zusätzlich konvex gewölbt ist.

Die Ausführungsformen nach den Fig. 10, 11 und 13 können gegebenenfalls so modifiziert sein, daß die nach ventral verlängerte Übergangszone 3′ nicht die ganze nach lateral vorstehende Osteotomiefläche am Pfannenfragment nach proximal abstützt, sondern nur deren lateralen Rand. Dazu ist der verlängerte Bereich 3′ gegenüber einem durch den Keilwinkel gegebenen Verlauf verschmälert, so daß die größte Breite der keilförmigen Übergangszone nicht am ventralen Rand 4 der Platte gelegen ist. Bei dieser Ausführungsform läßt sich eine Schraube nach dorsal nicht einsetzen.

Die erfindungsgemäßen Fixationsplatten können aus allen für diese Zwecke bekannten Materialien gebildet sein, insbesondere aus rostfreien Stahllegierungen und Titan.

Wie bereits erwähnt, soll die Darmbeinosteotomie im Sinne der Erhaltung einer möglichst hohen Kongruenz der Osteotomieflächen nach der Pfannendachverlagerung geführt werden. Als geometrisch ideales Modell ist hiebei eine sphärische Osteotomie anzusehen, die allerdings chirurgisch schwer zu verwirklichen ist. Es wurde nunmehr gefunden, daß eine gut praktikable Annäherung darin liegt, ebene Polygonalschnitte zu legen in Abhängigkeit vom durch Röntgendiagnose festgelegten Schwenkwinkel. Dazu werden entlang eines Kreisbogens ebene, möglichst gleichlange Annäherungsosteotomien jeweils in gleichem Winkel zueinander gelegt, wobei am Darmbein mindestens zwei ebene Teilosteotomien gelegt werden sollten.

Eine derartige Osteotomieführung ist aus Fig. 16 zu erkennen; die Darstellungsweise entspricht der der Figuren 1 und 2. Man erkennt, daß das Pfannenfragment im Dorsalbereich etwas nach medial gestellt ist, so daß im Bereich der nicht dargestellten Fixationsplatte die Osteotomiefläche am Pfannenfragment bezüglich der lateralen Osteotomiekante am Darmbein ventral nach lateral vor- und dorsal nach medial einspringt. Dies entspricht der Anwendung einer bezüglich des Kantenverlaufs an die ebene Schnittführung angepaßten Platte nach Fig. 5. Es sind deutlich die ebenen Osteotomien am Darmbein und am Sitzbein zu erkennen. Die Geometrie der Osteotomieführung nach Fig. 16 ist - in Ansicht von medial - aus den Fig. 14 und 15 zu erkennen.

Fig. 14 ist eine schematische Darstellung eines rechten Hüftgelenks in der Ebene des Darmbeins, wie sie röntgenologisch einfach erhalten werden kann. Dazu sollten folgende Bedingungen eingehalten werden: Röntgenquelle gelenkszentriert, Strahlrichtung etwa senkrecht zur Ebene der Ala ossis ilii, und etwa fluchtend mit Os pubis.

Diese Faux-Profil-Aufnahme dient der Festlegung der Führung der Osteotomien im Darmbein und Sitzbein Durch Röntgenaufnahmen des Gelenks in verschiedenen Raumansichten lassen sich vorher die ungefähren Schwenkwinkel für das Pfannenfragment festlegen, insbesondere der Hauptwinkel, der die Schwenkung nach ventrolateral betrifft. Dieser Winkel kann im Bereich von etwa 25 bis etwa 45° liegen, häufig im Bereich von 28 bis 38°, wobei schwere Hüftgelenksdysplasiefälle meist eine Schwenkung im Bereich von etwa 38° erfordern. Gleichfalls wird durch diese Aufnahmen die Lage des Pfannenzentrums bestimmt.

Eines der Näherungsverfahren zur Festlegung der Osteotomien im Darmbein erkennt man aus Fig.14. Zur Konstruktion wird die Incisura ischiadica major nach proximal verlängert; sie bildet die erste Schnittgerade. Weiterhin wird eine Tangente $T_1$ vom Pfannenzentrum PZ an die Projektion der Krümmung des Darmbeins anschließend an die Incisura ischiadica major und eine Tangente $T_2$ von dieser Projektion an die Projektiön des ventroproximalen Pfannenrands gelegt. Es hat sich gezeigt, daß der Schnittpunkt I der Verlängerung der Incisura ischiadica major mit der Tangente $T_1$ und der Schnittpunkt II mit der Tangente $T_2$ in guter Annäherung den ersten Eckpunkt des gesuchten Polygonzugs bilden, wobei der Schnittpunkt I den kleinen Schwenkwinkeln und der Schnittpunkt II den großen Schwenkwinkeln zugeordnet wird. In Fig. 6 ist eine Schwenkung um einen großen Schwenkwinkel dargestellt; es wird daher eine Gerade ausgehend vom Schnittpunkt II im Schwenkwinkel $\alpha$ von der Verlängerung der Incisura ischiadica major weggezogen bis zu dem Punkt, wo sie den Kreisbogen mit dem Radius $\overline{PZ\ II}$ schneidet. Die sich ergebende Osteotomielänge $\ell$ wird einerseits auf die Incisura ischiadica major zurückgeschlagen ($\ell_0$) und auf dem Kreisbogen weiter vorgeschlagen ($\ell_1$). Es ergibt sich der Polygonosteotomiezug im Darmbein und der Mindestabstand $l_0$ zu einem dorsalen Endpunkt E der Sitzbeinosteotomie. Die Endpunkte der Sitzbeinosteotomie am Foramen obturatum können innerhalb eines gewissen Bereichs frei gewählt werden.

In Fig. 15 ist das nach der Konstruktion gemäß Fig. 14 verschwenkte Pfannenfragment zu erkennen. Die Kongruenz ergibt sich aus der Gleichwinkeligkeit und der gleichen Länge $l = l_1$. Der im Bereich des Foramen ischiadicum nach der Schnittgeometrie verbleibende Knochenkeil ist in der Verlängerung von 1 ektomiert und die Distanz zum dorsalen Endpunkt $E_1$ der Sitzbeinosteotomie entsprechend größer als $l_0$ gewählt, sodaß auch in diesem Bereich die Osteotomieflächen aneinander anliegen.

Bei den polygonalen Osteotomien nach den Fig. 14 bis 16 sind die Fixationsplatten in ihrer Ausbildung natürlich entsprechend an den geraden Osteotomieverlauf anzupassen, insbesondere was die Kanten der keilförmigen Übergangszonen betrifft.

Wird als Schwenkzentrum nicht das Pfannenzentrum PZ gewählt, so kann je nach Indikation auch eine Beinverkürzung oder -verlängerung erzielt werden.

Zur praktischen Durchführung der Osteotomien kann es vorteilhaft sein, in Abhängigkeit von Beckengröße und Form Lehren- bzw. Schablonensätze vorzusehen, die die Osteotomiepolygone jeweils für die gewünschten Schwenkwinkel wiedergeben, so daß bei der Operation eine Elektrotom-Markierung am Darmbein und am Sitzbein jeweils einfach unter Anhalten der entsprechenden Schablone erfolgen kann. In ähnlicher Weise können vorgefertigte Fixierungsplattensätze vorgesehen sein.

## Patentansprüche

1. Knochenfixationsplatte zur Schraubfixierung des Pfannenfragments am Darmbein nach Beckenosteotomien mit Pfannendachschwenkung beim Menschen, dadurch gekennzeichnet, daß die Platte einen proximalen Abschnitt (1) zur im wesentlichen formschlüssigen Anlage an der Darmbeinschaufel und einen distalen Abschnitt (2) zur im wesentlichen formschlüssigen Anlage am Pfannenfragment aufweist, wobei die Abschnitte (1,2) in einem Winkel zueinander stehen, der im Bereich von 10 bis 50°, bevorzugt bei etwa 30°, liegt, und zur Bildung dieses Winkels zwischen den beiden Abschnitten (1,2) eine in Draufsicht keilförmige Übergangszone (3) zur Anlage an der Osteotomiefläche des Pfannenfragments und/oder zur Anlage an der Osteotomiefläche des Darmbeins ausgebildet ist.

2. Platte nach Anspruch 1, dadurch gekennzeichnet, daß die größte Breite der Übergangszone (3) am ventralen Rand (4) der Platte gelegen ist.

3. Platte nach Anspruch 1 oder 2, dadurch gekennzeichnet, daß an einen Abschnitt (3b) der Übergangszone (3) zur Anlage an der Osteotomiefläche des Pfannenfragments dorsal ein Abschnitt (3a) zur Anlage an der Osteotomiefläche des Darmbeins anschließt.

4. Platte nach einem der Ansprüche 1 bis 3, dadurch gekennzeichnet, daß die Abschnitte (1,2) der Platte an der dem Pfannenfragment zugewandten Seite zur Übergangszone (3) hin jeweils eine der Osteotomieführung angepaßte Kante, vorzugsweise im wesentlichen kreisbogenförmige Kanten (8,6), aufweisen.

5. Platte nach einem der Ansprüche 1 bis 4, dadurch gekennzeichnet, daß die Übergangszone (3) mit den beiden Abschnitten (1,2) der Platte jeweils stumpfe Winkel einschließt.

6. Platte nach einem der Ansprüche 1 bis 5, dadurch gekennzeichnet, daß die Platte räumlich gekrümmt ist, wobei insbesondere der proximale Abschnitt (1) zur Darmbeinschaufel hin im wesentlichen konvex und der distale Abschnitt (2) sowie die Übergangszone (3) zum Pfannenfragment hin im wesentlichen konkav ausgebildet sind.

7. Platte nach einem der Ansprüche 1 bis 6, dadurch gekennzeichnet, daß der proximale Abschnitt (1) in der Proximal-distalrichtung eine größere Erstreckung als der distale Abschnitt (2) aufweist, wobei Vorzugsweise die Übergangszone (3) und der distale Abschnitt (2) in der Ventral-dorsalrichtung gegenüber dem proximalen Abschnitt (1) eine größere Erstreckung aufweisen.

8. Platte nach einem der Ansprüche 1 bis 7, dadurch gekennzeichnet, daß die Platte eine größere Erstreckung in der Proximal-distalrichtung als in der Ventral-dorsalrichtung aufweist.

9. Platte nach einem der Ansprüche 1 bis 7, dadurch gekennzeichnet, daß die Platte eine größere Erstreckung in der Ventral-dorsalrichtung als in der Proximal-distalrichtung aufweist.

10. Platte nach einem der Ansprüche 1 bis 9, dadurch gekennzeichnet, daß die Platte mit in Proximal-distalrichtung verlängerten, unrunden Schraubensitzen mit asymmetrischer Ansenkung ausgestattet ist, wodurch die Schrauben im distalen Abschnitt (2) etwas nach distal und im proximalen Abschnitt (1) etwas nach proximal versetzt werden, so daß ein dynamischer Kompressionsdruck zwischen Darmbeinschaufel und Pfannenfragment erzielbar ist.

11. Platte nach einem der Ansprüche 1 bis 10, dadurch gekennzeichnet, daß der proximale Abschnitt (1) und der distale Abschnitt (2) in einem Winkel von 5 bis 60° gegeneinander gekippt sind (Fig. 4a, 4b).

12. Platte nach einem der Ansprüche 1 bis 11, dadurch gekennzeichnet, daß die Übergangszone (3) ferner
einen Medialisierungsabschnitt (MA) umfaßt, dessen Breite bis etwa 13 mm beträgt, der sich vom ventralen zum dorsalen Rand erstreckt.

13. Fixationssystem zur Fixierung des Pfannenfragments nach dreifachen Beckenosteotomien beim Menschen, bestehend aus einer Fixierungsplatte nach einem der Ansprüche 1 bis 12 mit entsprechenden Schrauben zur Fixierung des Pfannenfragments an der Darmbeinschaufel, sowie aus Befestigungsmitteln zur Fixierung des Pfannenfragments am Schambein, vorzugsweise einer Drahtcerclage über zwei Schrauben.

## Claims

1. A bone fixation plate for thread fixation of the acetabular fragment onto the ilium after pelvic osteotomies with displacement of the acetabular roof in humans, characterized in that the plate has a proximal portion (1) for substantially conforming abutment against the wing of the ilium and a distal portion (2) for substantially conforming abutment against the acetabular fragment, the portions (1,2) enclosing an angle of from 10 to 50°, preferably about 30°, and in that a transition zone (3) wedge-shaped in plan view for abutment against the osteotomy surface of the acetabular fragment and/or for abutment against the osteotomy surface of the ilium is formed between the two portions (1,2) in order to form this angle.

2. A plate according to claim 1, characterized in that the transition zone (3) has its largest width at the ventral rim (4) of the plate.

3. A plate according to claim 1 or claim 2, characterized in that a portion (3a) for abutment against the osteotomy surface of the ilium follows dorsally to a portion (3b) of the transition zone (3) for abutment against the osteotomy surface of the acetabular fragment.

4. A plate according to any of claims 1 to 3, characterized in that, on the side facing the acetabular fragment, the portions (1,2) of the plate each have an edge adapted to the osteotomy path, preferably substantially circularly arcuated edges (8, 6), towards the transition zone (3).

5. A plate according to any of claims 1 to 4, characterized in that the transition zone (3) encloses an obtuse angle with each of the two portions (1,2) of the plate.

6. A plate according to any of claims 1 to 5, characterized in that the plate is arcuate in space, in particular the proximal portion (1) being formed substantially convex towards the wing of the ilium and the distal portion (2) as well as the transition zone (3) substantially concave towards the acetabular fragment.

7. A plate according to any of claims 1 to 6, characterized in that the proximal portion (1) has a larger extension in proximal-distal direction than the distal portion (2), the transition zone (3) and the distal portion (2) preferably having a larger extension than the proximal portion (1) in ventral-dorsal direction.

8. A plate according to any of claims 1 to 7, characterized in that the plate has a larger extension in proximal-distal direction than in ventral-dorsal direction.

9. A plate according to any of claims 1 to 7, characterized in that the plate has a larger extension in ventral-dorsal direction than in proximal-distal direction.

10. A plate according to any of claims 1 to 9, characterized in that the plate is provided with extended, noncircular, asymmetrically countersunk screw fits, the screws thus being somewhat out of line towards the distal in the distal portion (2) and towards the proximal in the proximal portion (1), so that a dynamic compression force may be obtained between the wing of the ilium and the acetabular fragment.

11. A plate according to any of claims 1 to 10, characterized in that the proximal portion (1) and the distal portion (2) are tilted against each other at an angle of from 5 to 60° (Figs. 4a, 4b).

12. A plate according to any of claims 1 to 11, characterized in that furthermore, the transition zone (3) includes
a medialisation portion (MA) having a width of up to around 13 mm, which extends from the ventral to the dorsal rim.

13. A fixation system for fixation of the acetabular fragment following threefold pelvic osteotomies in humans, consisting of a fixation plate according to any of claims 1 to 12 with appropriate screws for fixation of the acetabular fragment to the wing of the ilium and attachment means for fixation of the acetabular fragment to the pubic bone, preferably a wire cerclage across two screws.

**Revendications**

1. Plaque à fixation d'os pour fixation à vis du fragment acétabulaire sur l'ilion après des ostéotomies pelviennes avec déplacement du toit de l'acétabule chez l'homme, caractérisée en ce que la plaque présente une section proximale (1) pour adjacence essentiellement fermée à l'aile iliaque et une section distale (2) pour adjacence essentiellement fermée au fragment acétabulaire, les sections (1,2) formant un angle de 10 à 50°, de préférence 30°, et en ce qu'une zone de transition (3), qui, vue d'en haut, est cunéiforme, pour adjacence à la surface d'ostéotomie du fragment acétabulaire et/ou pour adjacence à la surface d'ostéotomie de l'ilion est formée entre les deux sections (1,2) pour former cet angle.

2. Plaque selon la revendication 1, caracterisée en ce que la largeur la plus grande de la zone de transition (3) se trouve au bord (4) ventral de la plaque.

3. Plaque selon l'une des revendications 1 ou 2, caracterisée en ce qu'une section (3b) de la zone de transition (3) pour adjacence à la surface d'ostéotomie du fragment acétabulaire est suivie dorsalement d'une section (3a) pour adjacence à la surface d'ostéotomie de l'ilion.

4. Plaque selon l'une des revendications 1 à 3, caractérisée en ce que, au coté tourné vers le fragment acétabulaire, chacune des sections (1,2) de la plaque présente un chant adapté à la voie d'ostéotomie, de préférence des chants essentiellement en forme d'arc de cercle (8,6), vers la zone de transition (3).

5. Plaque selon l'une des revendications 1 à 4, caractérisée en ce que la zone de transition (3) forme un angle obtus avec chacune des deux sections (1,2) de la plaque.

6. Plaque selon l'une des revendications 1 à 5, caractérisée en ce que la plaque est arquée dans l'espace, en particulier la section proximale (1) étant formée essentiellement convexe vers l'aile iliaque et la section distale (2) aussi bien que la zone de transition (3) étant formées essentiellement concaves vers le fragment acétabulaire.

7. Plaque selon l'une des revendications 1 à 6, caracterisée en ce que, en direction proximale-distale, la section proximale (1) a une étendue plus grande que la section distale (2), la zone de transition (3) et la section distale (2) de préférance présentant une étendue plus grande en direction ventrale-dorsale que la section proximale (1).

8. Plaque selon l'une des revendications 1 à 7, caracterisée en ce que la plaque a une étendue plus grande en direction proximale-distale que en direction ventrale-dorsale.

9. Plaque selon l'une des revendications 1 à 7, caracterisée en ce que la plaque a une étendue plus grande en direction ventrale-dorsale que en direction proximale-distale

10. Plaque selon l'une des revendications 1 à 9, caractérisée en ce que la plaque est munie de logements de vis pas ronds, prolongés en direction proximale-distale et chanfreinés de façon asymmétrique, de sorte que les vis dans la section distale (2) sont déplacées un peu vers la distale et celles dans la section proximale (1) un peu vers la proximale, de manière que'on puisse obtenir une compression dynamique entre l'aile iliaque et le fragment acétabulaire.

11. Plaque selon l'une des revendications 1 à 10, caractérisée en ce que la section proximale (1) et la section distale (2) sont inclinées l'une vers l'autre à un angle de 5 à 60°. (figures 4a, 4b).

12. Plaque selon l'une des revendications 1 à 11, caractérisée en ce que de plus, la zone de transition (3) comporte
une section de medialisation (MA), dont la largeur monte jusqu'à 13 mm environ et qui s'étend du bord ventral au bord dorsal.

13. Système de fixation pour fixation du fragment acétabulaire après triples ostéotomies pelviennes chez l'homme, consistant d'une plaque à fixation selon l'une des revendications 1 à 12 avec vis appropriées pour la fixation du fragment acétabulaire sur l'aile iliaque, et des moyens d'attache pour la fixation du fragment acétabulaire sur le pubis, de préférence un cerclage en fil métallique sur deux vis.

Fig.1

Fig. 2

_Fig. 3_

_Fig. 4_

_Fig. 5_

OSIL

*Fig. 4a*

5

1

5°-60°

PF

2

5

*Fig. 4b*

1

4

3

2

OSIL

*Fig. 4c*

1

5

M

PF

2

5°-60°

*Fig. 4d*

1

MA

3

M

2

Fig. 9

Fig. 8

Fig. 7

Fig. 6

EP 0 253 796 B1

*Fig. 10*

*Fig. 11*

*Fig. 12*

*Fig. 13*

_Fig. 14_

# Fig. 15

Fig. 16

OSIL

PF

P

OSPU

OSIS